# EUROPEAN PATENT SPECIFICATION

(11) **EP 2 091 576 B1**
(45) Date of publication and mention of the grant of the patent: **02.01.2013**
(21) Application number: 07824613.9
(22) Date of filing: 16.11.2007
(51) Int. Cl.: A61L 9/14, B65D 83/14

(54) **DISPENSING DEVICE WITH REFILL CARTRIDGE AND JACKET ASSEMBLY**
SPENDEVORRICHTUNG MIT AUFFÜLLPATRONE UND MANTELANORDNUNG
DISPOSITIF DE DISTRIBUTION AVEC CARTOUCHE ET MANCHON

(30) Priority: 18.11.2006 GB 0623052
(43) Date of publication of application: 26.08.2009
(73) Proprietor: Reckitt Benckiser (UK) Limited, Slough, Berkshire SL1 3UH (GB)
(72) Inventor: ANDERSON, James, Hull HU8 7DS (GB); JIN, Wu, Hull HU8 7DS (GB); WOOLLEY, Simon, Hull HU8 7DS (GB); YE, Ivan, GuangDong 523900 (CN)
(74) Representative: Carlin, Robert George
(86) International application number: PCT/GB2007/004395
(87) International publication number: WO 2008/059275

(56) References cited:
- EP-A- 0 641 727
- EP-A- 1 076 014
- WO-A-2006/005962
- WO-A-2006/087515
- WO-A-2007/132140

## Description

The invention relates to a jacket assembly for a cartridge. Particularly, but not exclusively, the invention relates to a jacket assembly for an aerosol cartridge, for use in a device for dispensing a fluid for fragrancing, deodorising or sanitising air.

Dispensing devices, for example, air freshener dispensing devices as described in EP0641727, WO2006/087515, EP1076014, WO206/005962 are commonplace, particularly in domestic environments where they are used to create a pleasant fragrance or mask a bad odour. The devices are generally of two types, a stand-alone type which can be placed away from a mains socket and which may be electrically operated by means of an electrical lead or batteries, or the type which plugs directly in to a mains socket, known as a plug-in device. Usually, either one of these said types operates in combination with a fragrance refill cartridge. The refill cartridge may be gel-based in which fragrance in the gel emanates on contact with air, wick-based wherein a fragranced liquid is drawn up the wick and evaporates on exposure to the air, or alternatively the refill cartridge may be aerosol based wherein the fluid is dispensed under pressure.

Often, an aerosol refill cartridge is held in a holder or jacket assembly which is then inserted into a device. The jacket assembly provides a protective shield around the cartridge to minimise the risk of the cartridge being damaged, for example, during transit or storage. However, such jacket assemblies are often difficult to handle and are prone to being inserted into the device in an incorrect orientation. In so doing, there is an increased risk of the valve stem of the aerosol cartridge being damaged due to misalignment of the assembly.

It is an object to provide a jacket assembly for a cartridge which is easy to handle and the risk of misalignment in the device is minimised.

According to an aspect of the present invention there is provided a dispensing device assembly according to claim 1.

The jacket assembly of the invention may be provided together with a replacement cartridge or replacement cartridges may be provided separately. When provided together with a replacement cartridge, the jacket assembly provides a protective shield around the cartridge to minimise the risk of the cartridge being damaged, for example, during transit or storage. The jacket assembly and cartridge are then together inserted into a dispensing device, the jacket assembly having one or more and preferably one alignment member which ensures insertion in a correct orientation. The jacket assembly is readily removed without damage to the dispensing device to allow for replacement of the cartridge.

Preferably, the wall is substantially curvilinear in shape. Preferably, the wall is substantially cylindrical in shape. Preferably, the wall is circular in cross section.

The body has an opening for insertion of a cartridge in one end thereof.

The alignment member is a rectilinear projection of the wall of the body, parallel to and along the length of the longitudinal axis thereof. Preferably, the alignment member is substantially non deformable on insertion into a dispensing device. Preferably, the jacket assembly and alignment member are of the same cross section prior to, during and after insertion in a dispensing device.

Preferably, the alignment member is substantially rectangular in cross section, or at least is substantially rectangular in cross-section along three sides thereof.

Preferably, the alignment member extends at least half way along the length of the wall, preferably from the second end. Preferably, said member extends substantially two thirds of the length of the wall, preferably substantially between 2cm and 20cm, preferably substantially between 2cm and 10cm, most preferably substantially 4cm.

Preferably, the alignment member is substantially between 0.5cm and 5cm in width, preferably substantially between 1cm and 2cm, most preferably substantially 1.3cm. Preferably, the alignment member is substantially between 0.2cm and 1cm in depth, preferably substantially between 0.3cm and 0.8cm, most preferably substantially 0.5cm.

Provision of an alignment member allows the consumer to more easily grip the jacket assembly and guide said assembly into the device.

An abutment member is provided on the wall, the abutment member being adapted to abut against a ledge on an inner surface of a device. The abutment member is adjacent the alignment member. The abutment member lies transverse to the longitudinal axis of the alignment member, so that said members together form a T-shape. Preferably, a lower portion of the abutment member is coplanar with the second end of the body. Preferably, the alignment member and the abutment member form a one-piece moulding.

Preferably, shoulders extend from the body, preferably from the first end thereof. Preferably, at least one gripping portion is located on the shoulders, preferably being adapted to grip a neck section of a refill cartridge. Preferably, a plurality of gripping portions is provided. Preferably, the or each gripping portion is resiliently deformable and preferably tapers inwardly from the shoulders, to provide a snap fitting to hold the neck section in a locked position.

Preferably, a shielding means is located on the body, preferably at the first end. Preferably, the shielding means extends from the shoulders. Preferably, the shielding means comprises a plurality of upstanding members, preferably four said members, preferably equidistantly spaced apart. Preferably, said members are fixed to a collar which is adapted to surround an upper section of the refill cartridge.

Preferably, a nodule is located on at least one of the upstanding members. Preferably, the nodule is located towards an edge of said member. Preferably, the nodule projects upwardly from said member, preferably by a distance of substantially between 0.05cm to 0.5cm, preferably substantially between 0.1cm and 0.3cm, most preferably, substantially 0.2cm. Preferably, the nodule is adapted to abut against a switch on a surface of the device, preferably to cause the switch to move into an operating position.

Preferably, legs are located at the second end. Preferably, two legs are provided, each extending from the second end by substantially between 0.5cm and 3cm, preferably substantially between 1cm and 2cm, most preferably substantially 1.5cm. Preferably, the legs taper downwardly.

The jacket assembly is adapted to receive an aerosol refill cartridge, preferably said cartridge containing a fluid for fragrancing, deodorising or sanitising air.

The dispensing device comprises a cavity adapted to receive the jacket assembly, the cavity having a cut-out dimensioned to receive the alignment member. The cavity comprises the ledge against which the abutment member abuts, when the jacket assembly is in a loaded configuration.

Preferably, a switch is provided on the dispensing device, preferably in the cavity. Preferably, the switch is located towards a dispensing end of the device. Preferably, the switch is a push switch being sprung-loaded. Preferably, the switch is operable by the nodule on the jacket assembly when the jacket assembly is in the loaded configuration.

Preferably, a door is provided on the dispensing device. Preferably, the door is hingedly attached to said device. Preferably, the door is adapted to substantially close the cavity. Preferably, a closure member is provided on the door, being adapted to lock the door against the dispensing device, preferably when the jacket assembly is in the loaded configuration. Preferably, the closure member is resiliently deformable and preferably is received in a slot on said device.

Preferably, a spring is located in the door, preferably fixed to an inner face of the door, to project upwardly into the cavity. Preferably, the spring is substantially between 0.5cm and 1.5cm in length, most preferably substantially 0.9cm in length. Preferably, the spring travels a distance of substantially between 0.3cm to 1cm towards the inner face, most preferably substantially 0.6cm; this length is calculated from a starting length of 0.9cm. The spring force is preferably substantially between 35N and 50N , preferably substantially between 42N and 46N, most preferably substantially 44N.

According to a further aspect there is provided a dispensing device assembly comprising a dispensing device, a jacket assembly and a refill cartridge as hereinbefore described.

All of the features described herein may be combined with any of the above aspects, in any combination.

An embodiment of the invention will now be described, by way of example only, with reference to the accompanying drawings, wherein:
Figure 1 is a schematic perspective view of a jacket assembly;
Figure 2 is a schematic front view of a jacket assembly;
Figure 3 is a schematic side view of a jacket assembly;
Figure 4 is a further schematic side view of a jacket assembly;
Figure 5 is a schematic plan view of a jacket assembly;
Figure 6 is a schematic rear view of a jacket assembly;
Figure 7 is a schematic sectional view of a dispensing device and jacket assembly and refill cartridge according to the invention;
Figure 8 is a schematic rear view of a dispensing device with a jacket assembly and refill cartridge according to the invention;
Figure 9 is a schematic perspective view of a dispensing device with a jacket assembly and refill cartridge according to the invention;
Figure 10 is a schematic sectional view of a dispensing device comprising a jacket assembly and refill cartridge; and
Figures 11a and 11b show schematic sectional views of a dispensing device of Figure 10 in a first position and a second position respectively.

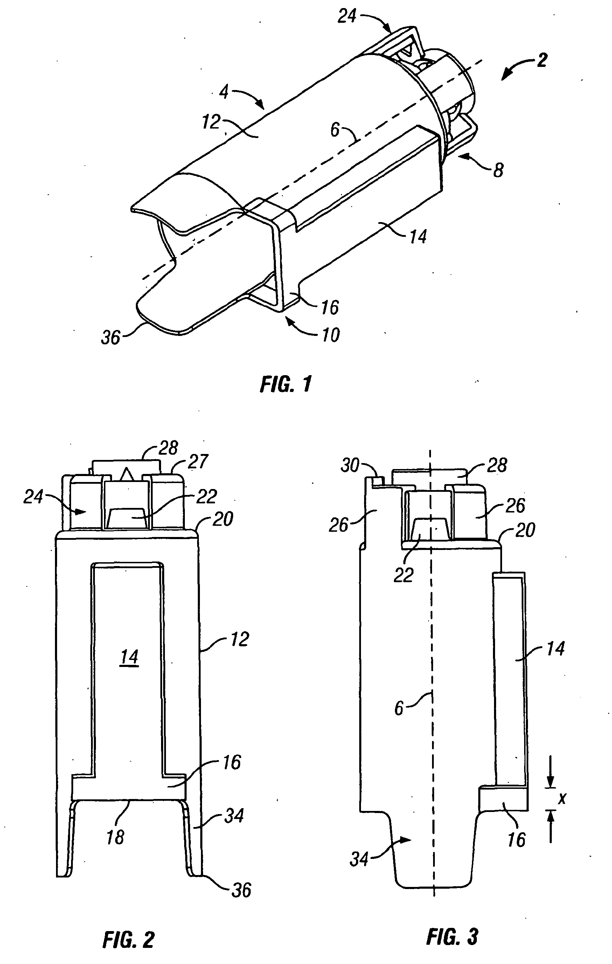

Figures 1 to 6 show a jacket assembly 2. The jacket assembly 2 comprises a body 4 which is generally circular in cross section and has a longitudinal axis 6. The body 4 comprises a first end 8, a second end 10 and a wall 12 therebetween.

An alignment member 14 is located on the wall 12. The alignment member 14 is generally rectangular in cross section and extends upwardly towards the first end 8, in parallel with the longitudinal axis 6. The alignment member 14 is approximately 4cm in length, 1.3cm in width and 0.5cm in depth.

An abutment member 16 is located on the wall 12. The abutment member 16 is generally rectangular in cross section having a length of approximately 2cm and a width of approximately 0.5cm. The abutment member 16 lies adjacent a lower section of the alignment member 14 and is transverse to the longitudinal axis 6 of the body 4 such that said members 14,16 together form a T-shape as shown most clearly in Figure 2. Said members 14,16 may be formed as a one-piece moulding. A lower portion 18 of the abutment member 16 is coplanar with the second end 10.

The body 4 comprises shoulders 20 which curve inwardly towards the first end 8. A plurality of gripping portions 22 extend from the shoulders 20 and taper inwardly of the body 4. Four gripping portions 22 are provided but it will be understood by the reader that any suitable number may be provided. The gripping portions 22 are resiliently deformable to snap fit around a neck of a refill cartridge placed into the jacket assembly 2.

Shielding means 24 also project upwardly from the shoulders 20. The shielding means 24 comprise a number of upstanding members 26, four being shown in the figures. The upstanding members 26 are equidistantly spaced apart and are positioned between each said gripping portion 22. A top part 27 of each of the upstanding members 26 is fixed to a collar 28. The collar 28 is circular in cross section and is dimensioned to allow only the valve stem of a refill cartridge to pass therethrough. The shielding means 24 forms a cage over a top part of a refill cartridge.

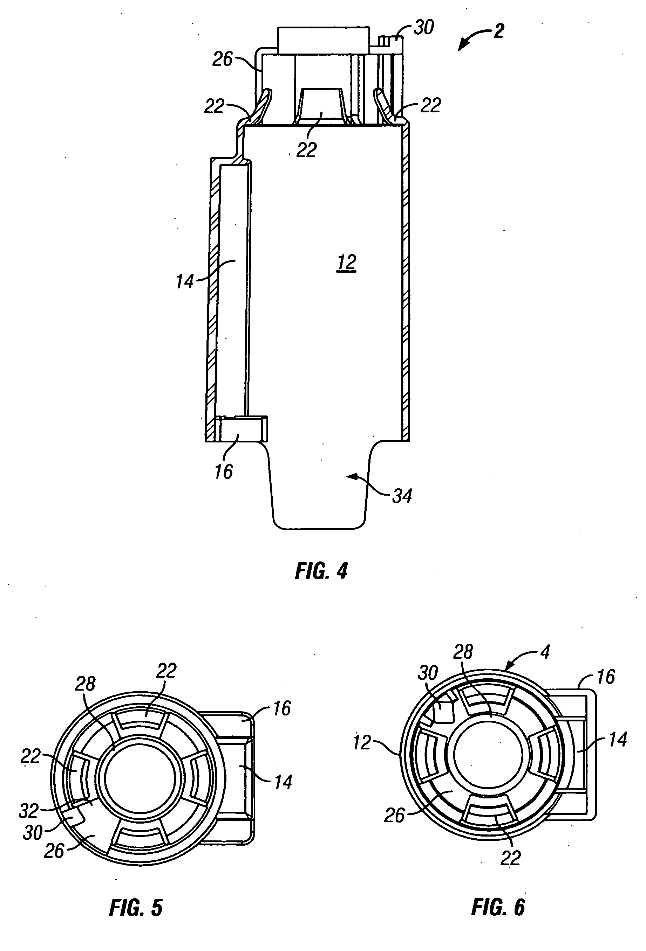

A nodule 30 is located on an upstanding member 26. Figure 5 shows a plan view of the jacket assembly 2 in which it can be seen that the nodule 30 is mounted adjacent an outer wall 32 of the upstanding member 26. The nodule 30 is generally rectangular in cross section and extends approximately 0.2cm from the upstanding member 26. The nodule 30 may be integrally moulded with the upstanding member 26.

Legs 34 are provided at the second end 10 of the body 4. The legs 34 extend approximately 1.5cm from the second end 10 and gently taper to feet 36. The feet 36 provide a platform on which the jacket assembly 2 rests when not in use in a dispensing device.

The jacket assembly 2 is configured to receive an aerosol refill cartridge, particularly a fragrance refill cartridge for an air freshener device.

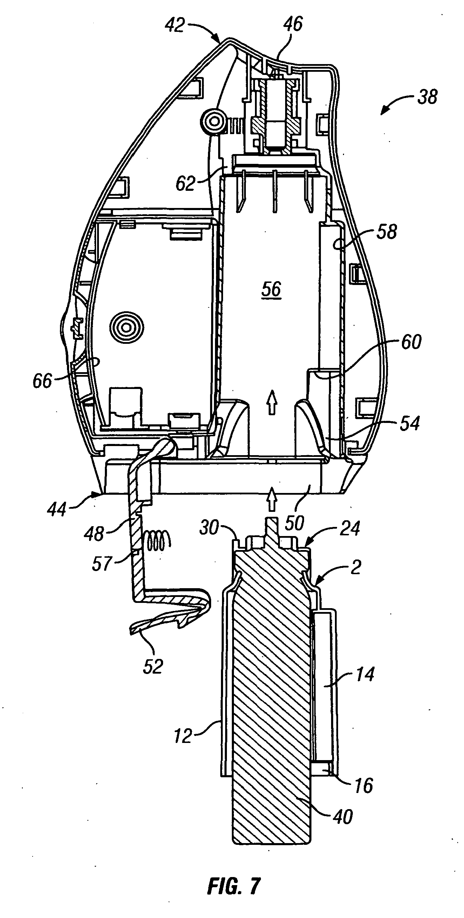

Figures 7 to 11 show a dispensing device 38 for a jacket assembly 2. The figures show the dispensing device 38 and jacket assembly 2 in use with a refill cartridge 40. The dispensing device 38 is generally tear-drop shaped having a top part 42 and a base 44. A fluid outlet 46 is provided in the top part 42 to allow fluid, for example, fragranced aerosol, to exit the device 38.

A hinged door 48 is located on the base 44 and is operable to close an opening 50 therein. The opening 50 is dimensioned to receive the jacket assembly 2. The hinged door 48 comprises a closure member 52 which is hook shaped and is resiliently deformable. The closure member 52 is received in a slot 54 in a cavity 56 of the device 38. A spring 57 is fixed to the hinged door 48 to project inwardly into the cavity 56 as shown in Figure 7. The spring 57 is approximately 0.9cm in length and travels a distance of 0.6cm in use. The force of the spring 57 is approximately 44N.

The cavity 56 extends from the opening 50. The cavity 56 is generally cylindrical having a cut-out 58 shaped to receive the alignment member 14. A ledge 60 is located at a lower end of the cut-out 58. The ledge 60 acts as a surface against which the abutment member 16 abuts when the jacket assembly 2 is placed inside the cavity 56. In so doing, the jacket assembly 2 is prevented from being inserted too far into the device 38.

A switch 62 is located on an upper surface of the cavity 56, on an outer section thereof. The switch 56 is generally rectangular in section and is operable to move from a first position to a second position by means of a spring attached thereto. The movement of the switch 56 is determined by the nodule 30 which pushes against the switch 56 when the jacket assembly 2 is in a loaded configuration.

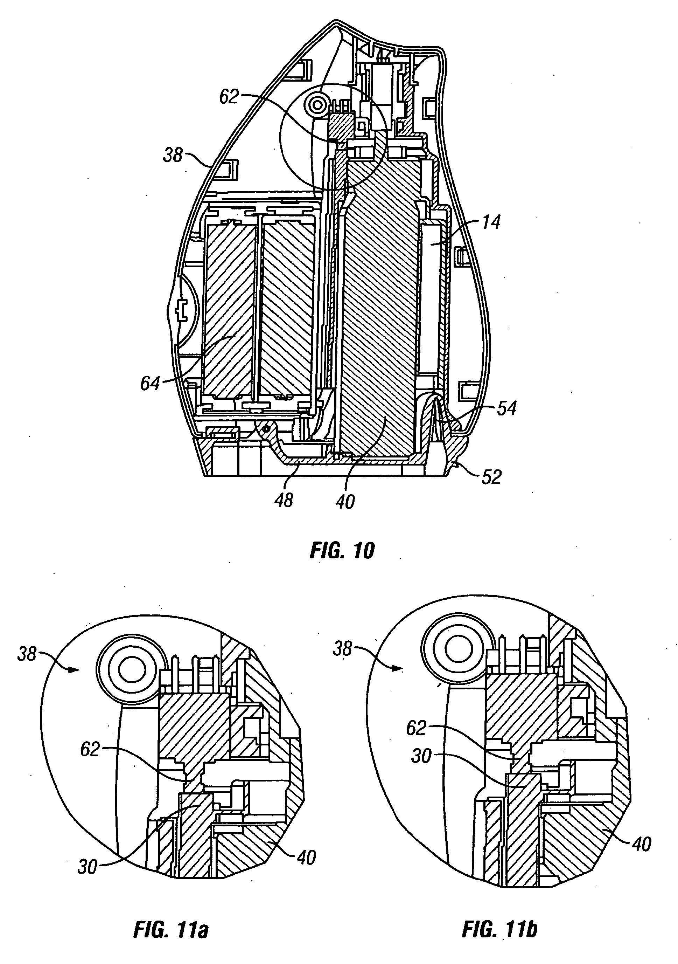

In the first position, the switch 56 extends into the cavity as shown in Figure 11a. In this position, the dispensing device 38 is inoperable to dispense fluid. When the switch 56 is moved to the second position as shown in Figure 11b, the switch 56 completes the circuitry to cause said device 38 to operate to dispense the fluid.

The dispensing device 38 may be mains operated or, as shown in the figures, can be operated by batteries 64 stored in a compartment 66.

In use, the user inserts the jacket assembly 2 into the device 38 through the opening 50. The alignment member 14 moves into the cut-out 58. The jacket assembly 2 is inserted until the abutment member 16 abuts against the ledge 60. The ledge 60 prevents the user from forcing the jacket assembly too far into the cavity 56. In this position, the nodule 30 aligns with the switch 62. When the door 48 is open, the nodule 30 does not push against the switch 56 and the switch 56 remains in the first position. When the user closes the door 48 and locks the closure member 52 into the slot 54, the spring 57 urges against the bottom of the refill cartridge 40. In so doing, the jacket assembly 2 pushes upwards and the nodule 30 urges against the switch 62.

The distance travelled by the spring 57 is approximately equivalent to the distance the nodule 30 moves to force the switch 62 into the second position. Therefore, when the door is closed 48, the dispensing device 38 is in an operating configuration.

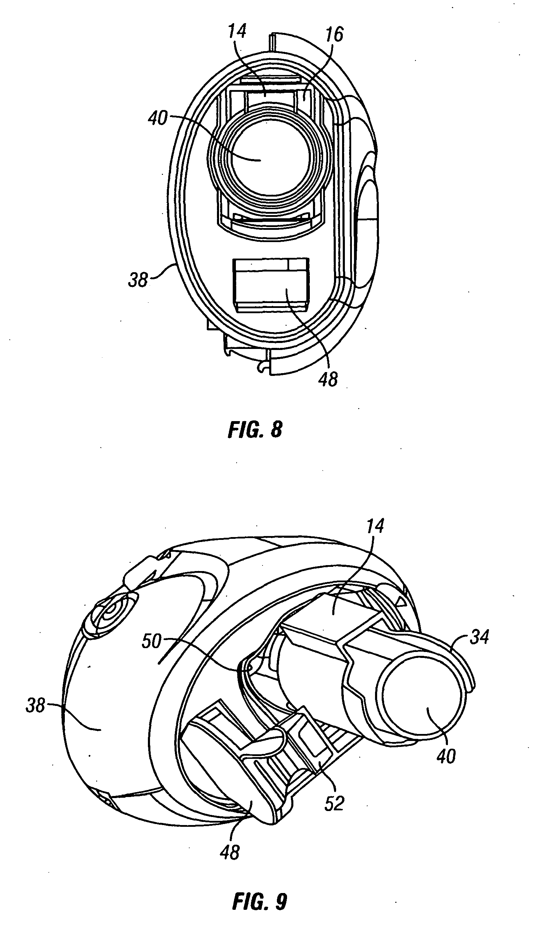

Due to the configuration of the alignment member 14 and the cavity 56, the jacket assembly 2 can only be inserted into the device 38 in one orientation. Further, due to the provision of the ledge 60, the jacket assembly 2 cannot be placed upside down into the device 38. In so doing, the user is prevented from damaging the device 38 and, particularly, the valve stem of the refill cartridge 40 due to misalignment of the cartridge 40. Figure 9 shows an example of an incorrectly positioned jacket assembly 2. It is shown that the alignment member 14 prevents full insertion of said assembly 2 into the cavity 56.

Advantageously, the shape of the alignment member 14 minimises the risk of said member 14 moving out of the cut-out 58. For example, if the alignment member 14 was curved, there would be an increased risk of slippage and movement. The rectilinear nature of the alignment member 14 is therefore highly advantageous. Further advantageously, the provision of the abutment member 16 and ledge 60 ensures that the jacket assembly 2 cannot be inserted too far into the device 38.

The alignment member 14 and the abutment member 16 guide the user when inserting the jacket assembly 2 into the device 38. In so doing, the user is less likely to damage the device 38, jacket assembly 2 or refill cartridge 40.

The reader's attention is directed to all papers and documents which are filed concurrently with or previous to this specification in connection with this application and which are open to public inspection with this specification.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment (s).

## Claims

1. A dispensing device assembly comprising a dispensing device (38), a jacket assembly (2) and refill cartridge (40) wherein the device (38) comprises a cavity (56) adapted to receive the jacket assembly (2), the cavity (56) having a cut-out (58) dimensioned to receive an alignment member and the cavity (56) further comprising a ledge (60) against which an abutment member abuts when the jacket assembly (2) is in a loaded configuration within the device (38);
and wherein the jacket assembly (2) grips a neck section of the refill cartridge (40) with at least one gripping portion (22) and the jacket assembly (2) further comprises a body (4) having a first end, a second end, a longitudinal axis therebetween and a wall extending between first and second ends, the wall being continuous in cross section and enclosing the longitudinal axis thereby providing an enclosure for the refill cartridge (40) and the body (4) having an opening for insertion of the cartridge (40) along the longitudinal axis, wherein the alignment member is located on the wall, the alignment member being rectilinear in shape and having a longitudinal axis which is parallel to the longitudinal axis of the body, **characterised in that** an abutment member is provided on the wall transverse to the longitudinal axis of the alignment member so that said members together form a T-shape, wherein the abutment member is adapted to abut against the ledge (60) on an inner surface of the device (38) to prevent further travel of the jacket assembly (2) into the device (38).

2. The dispensing device assembly according to claim 1, wherein the wall of the jacket assembly (2) is curvilinear in shape.

3. The dispensing device assembly according to claim 1, wherein the jacket assembly (2) has a shielding means (24) located on the body (4), the shielding means (24) comprising a plurality of upstanding members (26).

4. The dispensing device assembly according to claim 3, wherein a nodule (30) is located on at least one of the upstanding members of the jacket assembly (2).

5. The dispensing device assembly according to claim 4, wherein the nodule (30) projects from said member of the jacket assembly (2).

6. The dispensing device assembly according to claim 4 or claim 5, wherein the device (38) comprises a switch (62) provided in the cavity (56) located towards a dispensing end of the device (38), the switch (62) being a push switch and sprung loaded, and wherein the nodule on the jacket assembly (2) is adapted to abut against the switch (62) when said jacket assembly (2) is in a loaded configuration in the device (38) to cause the switch (62) in the device (38) to move into an operating position.

7. The dispensing device assembly according to claim 1, wherein the alignment member and the abutment member form a one-piece moulding.

## Patentansprüche

1. Spendevorrichtungsanordnung, aufweisend eine Spendevorrichtung (38), eine Mantelanordnung (2) und eine Auffüllpatrone (40), wobei die Vorrichtung (38) einen Hohlraum (56) aufweist, der dazu ausgelegt ist, die Mantelanordnung (2) aufzunehmen, wobei der Hohlraum (56) einen Ausschnitt (58) aufweist, der zum Aufnehmen eines Ausrichtungsglieds bemessen ist, und der Hohlraum (56) ferner einen Absatz (60) aufweist, an den ein Anstoßglied anstößt, wenn die Mantelanordnung (2) in einer belasteten Konfiguration innerhalb der Vorrichtung (38) ist;
und wobei die Mantelanordnung (2) einen Halsteilabschnitt der Auffüllpatrone (40) mit mindestens einem Greifabschnitt (22) greift und die Mantelanordnung (2) ferner einen Körper (4) mit einem ersten Ende, einem zweiten Ende, einer Längsachse dazwischen und einer Wand aufweist, die zwischen dem ersten und zweiten Ende verläuft, wobei die Wand im Querschnitt fortlaufend ist und die Längsachse einschließt, wodurch eine Einfassung für die Auffüllpatrone (40) und den Körper (4) mit einer Öffnung zur Einführung der Patrone (40) entlang der Längsachse vorgesehen ist, wobei sich das Ausrichtungsglied an der Wand befindet, wobei das Ausrichtungsglied rechteckig ist und eine Längsachse aufweist, die parallel zu der Längsachse des Körpers ist, **dadurch gekennzeichnet, dass** ein Anstoßglied derart quer verlaufend zu der Längsachse des Anstoßglieds an der Wand vorgesehen ist, dass die Glieder zusammen eine T-Form ausbilden, wobei das Anstoßglied dazu ausgelegt ist, an den Absatz (60) auf einer Innenfläche der Vorrichtung (38) zum Verhindern eines weiteren Laufwegs der Mantelanordnung (2) in die Vorrichtung (38) anzustoßen.

2. Spendevorrichtungsanordnung nach Anspruch 1, wobei die Wand der Mantelanordnung (2) eine krummlinige Form aufweist.

3. Spendevorrichtungsanordnung nach Anspruch 1, wobei die Mantelanordnung (2) ein Abschirmmittel (24) aufweist, das sich auf dem Körper (4) befindet, wobei das Abschirmmittel (24) mehrere aufrechte Glieder (26) aufweist.

4. Spendevorrichtungsanordnung nach Anspruch 3, wobei sich ein Knollen (30) auf mindestens einem der aufrechten Glieder der Mantelanordnung (2) befindet.

5. Spendevorrichtungsanordnung nach Anspruch 4, wobei der Knollen (30) von dem Glied der Mantelanordnung (2) vorsteht.

6. Spendevorrichtungsanordnung nach einem der Ansprüche 4 oder 5, wobei die Vorrichtung (38) einen Schalter (62) aufweist, der in dem Hohlraum (56) vorgesehen ist und zu einem Spendeende der Vorrichtung (38) hin angeordnet ist, wobei der Schalter (62) ein Druckschalter ist und federbelastet ist, und wobei der Knollen an der Mantelanordnung (2) dazu ausgelegt ist, an den Schalter (62) anzustoßen, wenn sich die Mantelanordnung (2) in einer belasteten Konfiguration in der Vorrichtung (38) befindet, um zu bewirken, dass sich der Schalter (62) in der Vorrichtung (38) in eine Betriebsposition bewegt.

7. Spendevorrichtungsanordnung nach Anspruch 1, wobei das Ausrichtungsglied und das Anstoßglied ein einstückiges Formteil ausbilden.

## Revendications

1. Ensemble de dispositif de distribution comprenant un dispositif de distribution (38), un ensemble de manchon (2) et une cartouche de remplissage (40), dans lequel le dispositif (38) comprend une cavité (56) prévue pour recevoir l'ensemble de manchon (2), la cavité (56) ayant une découpure (58) dimensionnée de manière à recevoir un organe d'alignement et la cavité (56) comprenant en outre un rebord (60) contre lequel vient buter un organe de butée lorsque l'ensemble de manchon (2) est dans une configuration chargée à l'intérieur du dispositif (38) ;
et dans lequel l'ensemble de manchon (2) saisit une section de col de la cartouche de remplissage (40) avec au moins une portion de préhension (22) et l'ensemble de manchon (2) comprend en outre un corps (4) ayant une première extrémité, une deuxième extrémité, un axe longitudinal entre elles et une paroi s'étendant entre les première et deuxième extrémités, la paroi étant continue en section transversale et enfermant l'axe longitudinal, en fournissant ainsi une enceinte pour la cartouche de remplissage (40), et le corps (4) ayant une ouverture pour l'insertion de la cartouche (40) le long de l'axe longitudinal, dans lequel l'organe d'alignement est placé sur la paroi, l'organe d'alignement étant de forme rectiligne et ayant un axe longitudinal qui est parallèle à l'axe longitudinal du corps, **caractérisé en ce qu'**un organe de butée est prévu sur la paroi transversale à l'axe longitudinal de l'organe d'alignement de telle sorte que lesdits organes forment ensemble une forme en T, l'organe de butée étant prévu pour buter contre le rebord (60) sur une surface intérieure du dispositif (38) pour empêcher une course supplémentaire de l'ensemble de manchon (2) dans le dispositif (38).

2. Ensemble de dispositif de distribution selon la revendication 1, dans lequel la paroi de l'ensemble de manchon (2) a une forme curviligne.

3. Ensemble de dispositif de distribution selon la revendication 1, dans lequel l'ensemble de manchon (2) a un moyen de protection (24) situé sur le corps (4), le moyen de protection (24) comprenant une pluralité d'organes dressés (26).

4. Ensemble de dispositif de distribution selon la revendication 3, dans lequel un nodule (30) est situé sur au moins l'un des organes dressés de l'ensemble de manchon (2).

5. Ensemble de dispositif de distribution selon la revendication 4, dans lequel le nodule (30) fait saillie depuis ledit organe de l'ensemble de manchon (2).

6. Ensemble de dispositif de distribution selon la revendication 4 ou 5, dans lequel le dispositif (38) comprend un commutateur (62) prévu dans la cavité (56) situé vers une extrémité de distribution du dispositif (38), le commutateur (62) étant un commutateur poussoir et étant précontraint par ressort, et le nodule sur l'ensemble de manchon (2) étant prévu pour buter contre le commutateur (62) lorsque ledit ensemble de manchon (2) est dans une configuration chargée dans le dispositif (38) pour amener le commutateur (62) dans le dispositif (38) à se déplacer dans une position de fonctionnement.

7. Ensemble de dispositif de distribution selon la revendication 1, dans lequel l'organe d'alignement et l'organe de butée forment un moulage d'une seule pièce.
